Europäisches Patentamt

European Patent Office  (11) Publication number: **0 136 475**

Office européen des brevets  **B1**

(12) **EUROPEAN PATENT SPECIFICATION**

(45) Date of publication of patent specification: **16.01.91**  (51) Int. Cl.⁵: **C 07 K 7/10, A 61 K 37/02**

(21) Application number: **84109312.3**

(22) Date of filing: **06.08.84**

(54) Novel growth hormone-releasing peptides and method of treating mammals therewith.

(30) Priority: **10.08.83 US 522067**

(43) Date of publication of application:
**10.04.85 Bulletin 85/15**

(45) Publication of the grant of the patent:
**16.01.91 Bulletin 91/03**

(84) Designated Contracting States:
**AT BE CH DE FR GB IT LI LU NL SE**

(56) References cited:
**EP-A-0 105 759**
**EP-A-0 107 890**
**EP-A-0 117 034**
**EP-A-0 121 764**
**EP-A-0 146 210**

**D.C. COY et al. (1985), Journal of Medical Chemistry, 28(2), 181-185**
**V.A. LANCE et al. (1984), B.B.R.C., 119(1), 265-272**

The file contains technical information submitted after the application was filed and not included in this specification

(73) Proprietor: **ADMINISTRATORS OF THE TULANE EDUCATIONAL FUND**
**St. Charles Avenue**
**New Orleans Louisiana 70118 (US)**

(72) Inventor: **Coy, David Howard**
**4319 Perrier Street**
**New Orleans Louisiana (US)**
Inventor: **Murphy, William Andrew**
**601 West Hall Avenue**
**Slidell Louisiana (US)**

(74) Representative: **Wächtershäuser, Günter, Dr.**
**Gewürzmühlstrasse 5**
**D-8000 München 22 (DE)**

Courier Press, Leamington Spa, England.

**Description**

Background of the Invention

The present invention relates to novel growth hormone-releasing peptides and method for increasing the release of growth hormone levels in mammals therewith.

Recently, growth hormone-releasing factors (GRF) of human pancreatic islet tumor origin (hpGRF) were isolated, characterized, and shown to process growth hormone(GH)-releasing activity in rat anterior pituitary *in vitro* and in vivo by (1) R. Guillemin, P. Brazeau, P. Böhlen, F. Esch, N. Ling, and W. B. Wehrenberg [Science, *218*, 585 (1982)] and (2) J. Spiess, J. Rivier, M. Thorner, and W. Vale [Biochemistry, *21*, 6037 (1982)]. Further, a synthetic hp GRF(1—29)—NH$_2$, an amidated fragment of the natural hp GRF, was reported to possess full intrinsic biological activity by Spiess *et al.* of reference (2). The European patent applications, publication numbers 0 117 134 and 0 121 764 also relate to novel growth hormone-releasing peptides.

## AMINO ACID SEQUENCE IN DESIGNATED PEPTIDES

hpGRF (1-44)NH$_2$

(Guillemin)   Tyr-Ala-Asp-Ala-Ile-Phe-Thr-Asn-Ser-Tyr-Arg-Lys-Val-Leu-Gly-Gln-Leu-Ser-Ala-Arg-
1

Lys-Leu-Leu-Gln-Asp-Ile-Met-Ser-Arg-Gln-Gln-Gly-Glu-Ser-Asn-Gln-Glu-Arg-Gly-Ala-
29                                                                                          40

Arg-Ala-Arg-Leu-NH$_2$;
44

hpGRF (1-40)

(Vale)   Tyr-Ala-Asp-Ala-Ile-Phe-Thr-Asn-Ser-Tyr-Arg-Lys-Val-Leu-Gly-Gln-Leu-Ser-Ala-Arg-
1

Lys-Leu-Leu-Gln-Asp-Ile-Met-Ser-Arg-Gln-Gln-Gly-Glu-Ser-Asn-Gln-Glu-Arg-Gly-Ala;
29                                                                                          40

hpGRF (1-29)NH$_2$

Tyr-Ala-Asp-Ala-Ile-Phe-Thr-Asn-Ser-Tyr-Arg-Lys-Val-Leu-Gly-Gln-Leu-Ser-Ala-Arg-
1

Lys-Leu-Leu-Gln-Asp-Ile-Met-Ser-Arg-NH$_2$.
29

EP 0 136 475 B1

Code for L-Amino Acid, per IUPAC-IUB Commission on Biochemical Nomenclature:
Biochemistry 11, 1726-1732 (1972)

| | | | |
|---|---|---|---|
| His = Histidine | Val = Valine | Tyr = Tyrosine | Gln = Glutamine |
| Ser = Serine | Phe = Phenylalanine | Arg = Arginine | Met = Methionine |
| Asp = Aspartic Acid | Thr = Threonine | Leu = Leucine | Ile = Isoleucine |
| Ala = Alanine | Asn = Asparagine | Lys = Lysine | Glu = Glutamic Acid |
| Gly = Glycine | | | |

EP 0 136 475 B1

Since these hpGRF peptides 29 to 44 amino acid units with their molecular weights ranging from 3,085 to 5,035 Daltons, it is desirable to increase their potency so that the dosages administered for practical purpose could be reduced for eliciting the release of suitable levels of GH in mammals. It is, therefore, an object of the present invention to provide more potent peptides which mimic hpGRF and are effective for increasing the release of growth hormone to suitable levels in mammals.

Summary of the Invention

Surprisingly, this objective is achieved by replacing the natural amino acids in positions 1, 2 and 3, as well as derivatizing the N-terminal amino acid residue in hpGRF 1 to 29, 1 to 40 and 1 to 44 as expressed below by the structural formulas (I), (II), and (III) respectively. The results are unexpected especially in view of reports by N. Ling and P. Brazeau [The Endocrine Society Program and Abstracts, 65th Annual Meeting, No. 295, June 8 to 10, 1983, at San Antonio, Texas] which indicate that Ac-Tyr[1] and D-Tyr[1] hpGRF analogs have low potency with respect to the natural peptide, and by J. Rivier et al. [Abstract 8th American Peptide Symposium, May 22—27, 1983, paper 10—B], who indicated for hpGRF (1—27) "some manipulation of the Tyr residue is compatible as long as a free amino terminus is conserved."

Description of the Invention

The polypeptides of the present invention are depicted by the structures of formulas (I), (II), and (III) below:

$$R^1-A-B-C-Ala-Ile-Phe-Thr-X-Ser-Tyr-Arg-Lys-Val-Leu-$$
$$1 \qquad\qquad\qquad 10$$

$$Gly-Gln-Leu-Ser-Ala-Arg-Lys-Leu-Leu-Gln-Asp-Ile-Y-$$
$$20$$

$$Ser-Arg-R^2$$
$$29$$

$$(I)$$

$$R^1-A-B-C-Ala-Ile-Phe-Thr-X-Ser-Tyr-Arg-Lys-Val-Leu-$$
$$1$$

$$Gly-Gln-Leu-Ser-Ala-Arg-Lys-Leu-Leu-Glu-Asp-Ile-Y-$$

$$Ser-Arg-Gln-Gln-Gly-Glu-Ser-Asn-Gln-Glu-Arg-Gly-Ala-R^2$$
$$29 \qquad\qquad\qquad\qquad\qquad\qquad 40$$

$$(II)$$

$$R^1-A-B-C-Ala-Ile-Phe-Thr-X-Ser-Tyr-Arg-Lys-Val-Leu-$$
$$1$$

$$Gly-Gln-Leu-Ser-Ala-Arg-Lys-Leu-Leu-Gln-Asp-Ile-Y-$$

$$Ser-Arg-Gln-Gln-Gly-Glu-Ser-Asn-Gln-Glu-Arg-Gly-Ala-$$
$$29$$

$$Arg-Ala-Arg-Leu-R^2$$
$$44$$

$$(III)$$

Wherein $R^1$ is hydrogen or $C_1$—$C_6$ straight- or branched-chain alkanoyl; $R^2$ is $NR^3R^4$ or $OR^3$, wherein $R^3$ and $R^4$ are selected from the group consisting of hydrogen and a straight- or branched-chain alkyl group containing 1—6 carbon atoms; A represents a member selected from tyrosyl, D-tyrosyl, or histidyl, B represents a member selected from alanyl, D-alanyl, C represents a member selected from aspartyl and D-aspartyl; X is asparaginyl or D-asparaginyl; Y is norleucyl or methionyl; with the proviso and the pharmaceutically acceptable salts thereof. * that either B and/ or C and/or X be a D amino acid;

Preferred compounds of the invention are represented by Formulas (I), (II), or (III) wherein $R^1$, $R^2$, $R^3$,

and R⁴ are selected from those described above; and A is selected from tyrosyl, _D_-tyrosyl, histidyl, _D_-histidyl; B is selected from alanyl, _D_-alanyl, leucyl, _D_-leucyl; C is selected from aspartyl, _D_-aspartyl, glutamyl and _D_-glutamyl; X is selected from asparaginyl or _D_-asparaginyl; Y is selected from norleucyl or methionyl; with the above-mentioned proviso; and the pharmaceutically acceptable salts thereof.

Another preferred group of compounds of the invention have the Formula (I) configuration, wherein R¹ is selected from hydrogen or $C_1$—$C_3$ alkanoyl; R² is NR³R⁴ or OR³; R³ and R⁴ are each selected from hydrogen and $C_1$—$C_3$ alkyl; A is selected from tyrosyl, _D_-tyrosyl or histidyl; B is alanyl or _D_-alanyl; C is aspartyl or _D_-aspartyl; X is asparaginyl or _D_-asparaginyl; Y is norleucyl or methionyl; with the above-mentioned proviso; and the pharmaceutically acceptable salts thereof.

The most preferred group of compounds of the invention have the Formula (I) configuration, wherein R¹ is selected from hydrogen or acetyl. R² is NH₂; A is selected from tyrosyl, _D_-tyrosyl or histidyl; B is alanyl or _D_-alanyl; C is aspartyl or _D_-aspartyl; X is asparaginyl or _D_-asparaginyl; und Y is norleucyl or methionyl; with the above-mentioned proviso; and the pharmaceutically acceptable salts thereof.

The term "pharmaceutically acceptable salts" as used herein refers to non-toxic alkali metal, alkaline earth metal, ammonium, organoammonium and metallic salts commonly used in the pharmaceutical industry. These salts include, but are not limited to, the sodium, potassium, lithium, calcium, magnesium, zinc, ammonium and trimethylammonium salts which are prepared by methods well known in the art. The term also includes non-toxic acid addition salts such as hydrochloride, hydrobromide, acetate, phosphate, sulfate, citrate, laurate, stearate, pamoate, and oleate, but are not limited to them. These acid addition salts are also prepared by methods well known in the art.

In keeping with the standard nomenclature, abbreviations for chiral amino acid residues used in the present specification and claims are as follows:

| Abbreviation | Name |
|---|---|
| His | L-histidyl |
| Ser | L-serinyl |
| Asp | L-aspartyl |
| D-Asp | D-aspartyl |
| Ala | L-alanyl |
| D-Ala | D-alanyl |
| Val | L-valinyl |
| Phe | L-phenylalanyl |
| Thr | L-threonyl |
| Asn | L-asparaginyl |
| D-Asn | D-asparaginyl |
| Tyr | L-tyrosinyl |
| Arg | L-arginyl |
| Leu | L-leucyl |
| D-Leu | D-leucyl |
| Nle | L-norleucyl |
| Lys | L-lysyl |
| Gln | L-glutaminyl |
| Met | L-methionyl |
| Ile | L-isoleucyl |
| Glu | L-glutamyl |
| D-Glu | D-glutamyl |
| D-Tyr | D-tyrosyl |
| D-His | D-histidyl |

6

| Abbreviation | Name |
|---|---|
| D-Phe | D-phenylalanyl |
| (4-Cl)Phe | L-4-chlorophenylalanyl |
| D-(4-Cl)Phe | D-4-chlorophenylalanyl |
| (4-Br)Phe | L-4-bromophenylalanyl |
| D-(4-Br)Phe | D-4-bromophenylalanyl |
| (4-F)Phe | L-4-fluorophenylalanyl |
| D-(4-F)Phe | D-4-fluorophenylalanyl |
| (4-MeO)Phe | L-4-methoxyphenylalanyl |
| D-(4-MeO)Ph3 | D-4-methoxyphenylalanyl |
| (4-$\emptyset$CH$_2$O)Phe | L-4-benzloxyphenylalanyl |
| D-(4-$\emptyset$CH$_2$O)Phe | D-4-benzloxyphenylalanyl |
| Trp | L-tryptophyl |
| D-Trp | D-tryptophyl |
| (5-F)Trp | L-5-fluorotryptophyl |
| D-(5-F)Trp | D-5-fluorotryptophyl |
| (5-Cl)Trp | L-5-chlorotryptophyl |
| D-(5-Cl)Trp | D-5-chlorotryptophyl |
| (5-Br)trp | L-5-bromotryptophyl |
| D-(5-Br)Trp | D-5-bromotryptophyl |
| (5-MeO)Trp | L-5-methoxytryptophyl |
| D-(5-MeO)Trp | D-5-methoxytryptophyl |
| (5-Me)Trp | L-5-methyltryptophyl |
| D-(5-Me)Trp | D-5-methyltryptophyl |

Unless otherwise specified, the amino acid residues that are named herein without the prefix L will refer to the naturally occurring absolute configuration L. The $R^1$ group refers to the substituent on the N-terminus amino acid (position 1) of the peptide according to standard nomenclature.

Other abbreviations used in the present specification are:

| | | |
|---|---|---|
| Fmoc | = | fluorenylmethyloxycarbonyl |
| Boc | = | t-butyloxycarbonyl |
| Tos | = | p-toluenesulfonyl |
| hplc | = | high-performance liquid chromatography |
| tlc | = | thin-layer chromatography |
| TFA | = | trifluoroacetic acid |
| Ac | = | acetyl |

Solid-phase synthesis of the Formulas (I), (II), and (III) peptides can be carried out on a Beckman 990 automatic peptide synthesizer. Preparative HPLC can be performed on a thick-walled glass column (2.5 ×

7

45 cm) containing Whatman LRP—1 reverse phase packing ($C_{18}$ silica 13—20 μm) pumped with Fluid Metering Company pump and pulse damper. Amino acid analyses can be run on a Beckman 119 CL analyzer and processed with a System AA computing integrator.

Amino acid derivatives utilized in the preparation of the compounds of the present invention are available from several chemical supply houses including: Bachem, Inc., Torrance, California, and Chemical Dynamics, Inc., Plainfield, New Jersey.

The peptides having the Formulas (I), (II), and (III) configurations can be conveniently prepared by standard solid-phase techniques; for example, the C-terminal protected amino acid can be attached to a chloromethyl resin, a hydroxymethyl resin, a benzhydrylamine (BHA) resin, or a p-methylbenzyl-hydrylamine (p-Me-BHA) resin. One such chloromethyl resin is sold under the trade name Bio-Beads SX—1 by Bio Rad Laboratories, Richmond, California. The preparation of the hydroxymethyl resin is described by Bodansky et al., Chem. Ind. (London) 38, 1957 (1966). The BHA resin has been described by Pietta and Marshall, Chem. Commun. 650 (1970) and commercially available from Bachem, Inc., Torrance, California.

According to an embodiment of the invention, the peptides of Formulas (I), (II), and (III) are prepared by means of solid-phase peptide synthesis by standard procedures, although it may also be prepared by treatment of the peptide-resin with ammonia to give the desired side-chain protected amide or with an alkylamine to give a side-chain protected alkylamide or dialkylamide.

The α-amino protecting group is Fmoc, and the side-chain protecting group is t-Bu instead of benzyl for the appropriate amino acid when the chloromethyl or hydroxymethyl resin is used.

Side-chain protection can then be removed in the usual fashion by treatment with HF to give the free peptide amides, alkylamides, or dialkylamides.

In preparing the esters of this invention, the resins used to prepare the acids of Formulas (I), (II), and (III) ($R^2$ = OH) can be employed, and the side-chain protected peptide can be cleaved with a base and an appropriate alcohol, i.e., methanol. Side-chain protecting groups can then be removed in the usual fashion by treatment with HF to obtain the desired ester.

The solid-phase procedure discussed above is well known in the art and has been essentially described by Stewart and Young, Solid Phase Peptide Synthesis, Freeman and Company, San Francisco, California (1969).

FLOW DIAGRAM I

Preparation of [D-Ala$^2$]-hpGRF(1-29)-NH$_2$

BHA Resin + Boc-Arg(Tos)
$\left.\begin{array}{l} \cdot \;| \;\;(CH_3)_2CH-N=C=N-CH(CH_3)_2 \\ \;\;\;\;\Big\downarrow CH_2Cl_2 \end{array}\right\} > A$

Boc-Arg(Tos)-BHA Resin

$\left.\begin{array}{l} \text{(a) } CH_2Cl_2 \text{ wash} \\ \text{(b) } 33\% \text{ TFA/}CH_2Cl_2, \text{ twice} \\ \text{(c) } CH_2Cl_2 \text{ wash} \\ \text{(d) } EtOH \text{ wash} \\ \text{(e) } CH_2Cl_2 \text{ wash} \\ \text{(f) } Et_3N/CHCl_3, \text{ twice} \\ \text{(g) } CH_2Cl_2 \text{ wash} \end{array}\right\} > B$

Boc-Arg(Tos)-BHA Resin

(1) Boc-Ser($\emptyset CH_2$), A, B
(2) Boc-Met, A, B
(3) Boc-Ile, A, B
(4) Boc-Asp($\emptyset CH_2$), A, B
(5) Boc-Gln, A, B
(6) Boc-Leu, A, B
(7) Boc-Leu, A, B
(8) Boc-Lys(4-Cl$\emptyset CH_2OCO$), A, B
(9) Boc-Arg(Tos), A, B
(10) Boc-Ala, A, B
(11) Boc-Ser($\emptyset CH_2$), A, B
(12) Boc-Leu, A, B
(13) Boc-Gln, A, B
(14) Boc-Gly, A, B
(15) Boc-Leu, A, B
(16) Boc-Val, A, B
(17) Boc-Lys(4-Cl$\emptyset CH_2OCO$), A, B
(18) Boc-Arg(Tos), A, B
(19) Boc-Tyr(4-Br$\emptyset CH_2OCO$), A, B

FLOW DIAGRAM I (Continued)

    (20) Boc-Ser(ØCH₂), A, B,
            MeOH wash, cycle B
    (21) Boc-Asn, A, B
    (22) Boc-Thr(ØCH₂), A, B
    (23) Boc-Phe, A, B
    (24) Boc-Ile, A, B
    (25) Boc-Ala, A, B
    (26) Boc-Asp(ØCH₂), A, B
    (27) Boc-D-Ala, A, B
    (28) Boc-Tyr, A, B

D-Ala²hpGRF(1-29)-BHA Resin

    (a) HF, dimethylsulfide,
            p-cresol
    (b) Et₂O wash
    (c) HOAc, Sephadex G-50 column
            chromatography
    (d) Lyophilize
    (e) Chromatography:octadecyl-
            silane silica, 15-50%
            CH₃CN/H₂O/0.1% TFA/80 psi
    (f) Lyophilize

[D-Ala²]-hpGRF(1-29)-NH₂

To prepare formula (II) analogs, the C-terminal protected amino acid Boc-alanine is attached to the desired resin as described for preparing formula (I) peptides; likewise, to prepare formula (III) analogs, the C-terminal protected amino acid Boc-leucine is attached to the desired resin. The subsequent amino acid groups are then sequentially coupled to the solid phase in the manner described in Flow Diagram I.

To prepare a N-terminal alkanoyl [R¹ of (I), (II), and (III)] peptide, the peptide bonded to the resin is allowed to stir in 5 to 20% solutions of the appropriate acid anhydride in CH₂Cl₂ containing triethylamine or other standard acid accepting bases for 20 to 120 minutes at room temperature. Subsequently, the standard reagents for cleaving the peptide from the resin is used to obtain the desired peptide of formulas (I), (II), or (III).

Thus, by the above-mentioned procedure, the following peptides are prepared:

[N-Acetyl]-hpGRF(1-29)-$NH_2$

$\underline{D}$-Tyr$^1$]-hpGRF(1-29)-$NH_2$

[N-Acetyl-$\underline{D}$-Tyr$^1$]-hpGRF(1-29)-$NH_2$

[N-Acetyl,$\underline{D}$-Ala$^2$]-hpGRF(1-29)-$NH_2$

[$\underline{D}$-Asp$^3$]-hpGRF(1-29)-$NH_2$

[N-Acetyl,$\underline{D}$-Asp$^3$]-hpGRF(1-29)-$NH_2$

[$\underline{D}$-Tyr$^1$,$\underline{D}$-Ala$^2$]-hpGRF(1-29)-$NH_2$

[N-Acetyl-$\underline{D}$-Tyr$^1$,$\underline{D}$-Ala$^2$]-hpGRF(1-29)-$NH_2$

[His$^1$,$\underline{D}$-Ala$^2$]-hpGRF(1-29)-$NH_2$

[N-Acetyl-His$^1$,$\underline{D}$-Ala$^2$]-hpGRF(1-29)-$NH_2$

[$\underline{D}$-Tyr$^1$,$\underline{D}$-Ala$^2$,$\underline{D}$-Asp$^3$]-hpGRF(1-29)-$NH_2$

[N-Acetyl-$\underline{D}$-Tyr$^1$,$\underline{D}$-Ala$^2$,$\underline{D}$-Asp$^3$]-hpGRF(1-29)-$NH_2$

[His$^1$,$\underline{D}$-Ala$^2$,$\underline{D}$-Asp$^3$]-hpGRF(1-29)-$NH_2$

[N-Acetyl-His$^1$,$\underline{D}$-Ala$^2$,$\underline{D}$-Asp$^3$]-hpGRF(1-29)-$NH_2$

[$\underline{D}$-Asn$^8$]-hpGRF(1-29)-$NH_2$

[$\underline{D}$-Ala$^2$,Nle$^{27}$]-hpGRF(1-29)-$NH_2$

[His$^1$,$\underline{D}$-Ala$^2$,Nle$^{27}$]-hpGRF(1-29)-$NH_2$

[$\underline{D}$-Ala$^2$,$\underline{D}$-Asn$^8$,Nle$^{27}$]-hpGRF(1-29)-$NH_2$

[$\underline{D}$-Asp$^3$,$\underline{D}$-Asn$^8$,Nle$^{27}$]-hpGRF(1-29)-$NH_2$

[$\underline{D}$-Ala$^2$,$\underline{D}$-Asp$^3$,$\underline{D}$-Asn$^8$,Nle$^{27}$]-hpGRF(1-29)-$NH_2$

[$\underline{D}$-His$^1$]-hpGRF(1-29)-$NH_2$

[N-Acetyl-$\underline{D}$-His$^1$]-hpGRF(1-29)-$NH_2$

[$\underline{D}$-Leu$^2$]-hpGRF(1-29)-$NH_2$

[N-Acetyl,$\underline{D}$-Leu$^2$]-hpGRF(1-29)-$NH_2$

[Asp$^3$]-hpGRF(1-29)-$NH_2$

[N-Acetyl,Asp$^3$]-hpGRF(1-29)-$NH_2$

[$\underline{D}$-Glu$^3$]-hpGRF(1-29)-$NH_2$

[N-Acetyl,$\underline{D}$-Glu$^3$]-hpGRF(1-29)-$NH_2$

[$\underline{D}$-Ala$^2$]-hpGRF(1-40)-$NH_2$

[$\underline{D}$-Ala$^2$]-hpGRF(1-44)-$NH_2$

[N-Acetyl,$\underline{D}$-Ala$^2$]-hpGRF(1-40)-$NH_2$

[N-Acetyl,$\underline{D}$-Ala$^2$]-hpGRF(1-44)-$NH_2$

---

Accordingly, the present invention includes pharmaceutical compositions comprising at least one of the peptides of formulas (I), (II), or (III) as an active ingredient, in association with a pharmaceutical carrier or diluent for use in stimulating growth-hormone release in mammals as follows:

EP 0 136 475 B1

R¹-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-R²;

(I)

or

R¹-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-Gln-Gln-Gly-Glu-Ser-Asn-Gln-Glu-Arg-Gly-Ala-R²

(II)

or

R¹-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-Gln-Gln-Gly-Glu-Ser-Asn-Glu-Glu-Arg-Gly-Ala-Arg-
Ala-Arg-Leu-R²;

(III)

wherein $R^1$ is hydrogen or $C_1$—$C_6$ straight- or branched- chain alkanoyl; $R^2$ is $NR^3R^4$ or $OR^3$; $R^3$ and $R^4$ are selected from the group consisting of hydrogen and a straight- or branched-chain alkyl group containing one to six carbon atoms; A represents a member selected from tyrosyl, D-tyrosyl and histidyl, B represents a member selected from alanyl and D-alanyl, C represents a member selected from aspartyl and D-aspartyl; X represents asparaginyl or D-asparaginyl; Y represents norleucyl or methionyl; with the proviso that either B and/or C and/or X be a D amino acid; and the pharmaceutically acceptable salts thereof.

In practice, it has been found that the Formulas (I), (II), and (III) compounds of the present invention are effective for increasing the release of growth hormone in mammals when administered thereto in an amount sufficient to provide said-treated mammals with from 0.000001 to 0.1 mg/kg of mammalian body weight/day of said Formulas (I), (II), or (III) compound.

Preferred peptides for increasing release of growth hormone in mammals have a structure selected from Formulas (I), (II), or (III) above, wherein $R^1$, $R^2$, $R^3$ and $R^4$ are selected from those described above, A is tyrosyl, D-tyrosyl or histidyl, B is alanyl or D-alanyl; C is aspartyl or D-aspartyl; X is asparaginyl or D-asparaginyl; Y is norleucyl or methionyl; with the above-mentioned proviso; and the pharmaceutically acceptable salts thereof.

Another preferred group of compounds of the present invention effective for increasing growth hormone-release in mammals have the structure illustrated by Formula (I) wherein $R^1$ is hydrogen or $C_1$—$C_3$ alkanoyl; $R^2$ is $NR^3R^4$ or $OR^3$; $R^3$ and $R^4$ are each hydrogen or a straight- or branched-chain or $C_1$—$C_3$ alkyl; A is tyrosyl, D-tyrosyl, or histidyl; B is alanyl or D-alanyl; C is aspartyl or D-aspartyl; X is asparaginyl or D-asparginyl; Y is norleucyl or methionyl; with the above-mentioned proviso; and the pharmaceutically acceptable salts thereof.

The most preferred group of compounds of the invention have the Formula (I) configuration, wherein $R^1$ is selected from hydrogen or acetyl; $R^2$ is $NH_2$; A is selected from tyrosyl, D-tyrosyl or histidyl; B is alanyl or D-alanyl; C is aspartyl or D-aspartyl; X is asparaginyl or D-asparaginyl; and Y is norleucyl or methionyl; with the above-mentioned proviso; and the pharmaceutically acceptable salts thereof.

These peptides are useful for treatment for symptoms related to growth hormone deficiencies, for increasing wool growth, for increasing rate of growth of meat-producing animals, for improving carcass quality in meat-producing animals (i.e., more protein and less fat), for improving feed efficiency in meat-producing animals and dairy cows, for increasing milk production in dairy herds, and in healing wounds.

In order to facilitate a further understanding of the invention, the following examples are presented primarily for the purpose of illustrating certain more specific details thereof. The invention is not to be deemed limited thereby except as defined in the claims.

Example 1

Protected human pancreatic growth hormone-releasing factor (9—29)-benzhydrylamine resin

Benzhydrylamine polystrene resin (commercially available from Bachem, Inc., Torrance, California) (6.0 g, 3.00 mmol) in the chloride ion form is placed in the reaction vessel of a Beckman 990 automatic

12

peptide synthesizer programmed to carry out the following work-wash cycle: (a) $CH_2Cl_2$; (b) 33% trifluoroacetic acid in $CH_2Cl_2$ (two times for one and 25 minutes each); (c) $CH_2Cl_2$; (d) $C_2H_5OH$; (e) $CH_2Cl_2$; (f) 10% $(C_2H_5)_3N$ in $CHCl_3$ (two times for two minutes each); and (g) $CH_2Cl_2$.

The neutralized resin is stirred with *t*-butyloxycarbonyl(Boc)-N-tosyl-l-arginine [Boc-Arg(Tos)] and diisopropylcarbodiimide (6 mmol) in $CH_2Cl_2$ for one hour, and the resulting amino acid resin is then cycled through the steps (a) through (g) in the above wash program. The following *L*-amino acids (3 mmol) are then coupled successively by the same reaction cycle: Boc-Ser(benzyl), Boc-Met, Boc-Ile, Boc-Asp(benzyl), Boc-Gln, Boc-Leu, Boc-Leu, Boc-Lys(4-chlorocarbenzoxy), Boc-Arg(Tos), Boc-Ala, Boc-Ser(benzyl), Boc-Leu, Boc-Gln, Boc-Gly, Boc-Leu, Boc-Val, Boc-Lys(4-chlorocarbenzoxy), Boc-Arg(tosyl), Boc-Tyr(4-bromocarbenzoxy) and Boc-Ser(benzyl), except that Boc-Gln was coupled in the presence of 1-hydroxybenzotriazole (6 mmol) in dimethylformamide solution.

The completed peptide-benzhydrylamine resin, with the N-terminal Boc group removed, was then washed with $CH_3OH$ and air dried to give 11.79 g of material.

## Example 2
Preparation of protected *D*-tyrosine-1-human pancreatic growth hormone-releasing factor (1—29)-benzhydrylamine resin

Peptide benzhydrylamine resin (0.98 g, 0.25 mmol) containing the 9—29 residues of the peptide as described in Example 1 is subjected to the work-wash cycle also described in Example 1. The neutralized resin is stirred with Boc-*L*-asparagine (0.75 mmol), diisopropylcarbodiimide (0.75 mmol), and 1-hydroxybenzotriazole (0.75 mmol) in dimethylformamide.

The following amino acid derivatives (0.75 mmol) are then coupled successively by the same treatment cycle described in Example 1: Boc-Thr(benzyl), Boc-Phe, Boc-Ile, Boc-Ala, Boc-Asp(benzyl), Boc-Ala, and Boc-D-Tyr. The completed 1—29 peptide resin is then cycled through the standard work-wash program described in Example 1 in oder to remove the N-terminal Boc group.

## Example 3
Preparation of *D*-tyrosine-l-human pancreatic growth hormone-releasing(1—29)-amide [(*D*-Tyr[1])-hpGRF(1—29)-$NH_2$]

A mixture of the 1—29 peptide resin described in Example 2 (0.75 mmol) and a solution of hydrogen fluoride (10 mL), dimethylsulfide (26 mL), and *p*-cresol (4 mL) are stirred at 0°C for 75 minutes. Excess reagents are then rapidly evaporated under a stream of dry nitrogen and hydrogen fluoride (35 mL) is added and the mixture stirred for a further 45 minutes at 0°C. Excess hydrogen fluoride is evaporated under nitrogen, and the resin plus free peptide are washed free of *p*-cresol with a large volume of diethyl ether.

The peptide is extracted into 50% acetic acid solution and applied to a column (2.5 × 95 cm) of Sephadex G—50 which is eluted with 2 M acetic acid. Eluant is monitored at 280 nM and fractions containing a major uv-absorbing peak are pooled and lyophilized. A solution of the lyophilized powder is eluted on a column (1.5 × 45 cm) of octadecylsilane silica having a mesh size of 15—20 M and pore size of 300 Å (purchased from Vydac, Hesperia, California). A linear elution gradient of 15—50% acetonitrile on 0.1% trifluoroacetic acid solution was employed at a pumping pressure of about 80 psi. Emerging fractions are monitored at 280 nm and are each examined by analytical hplc at a wave length of 215 nm in order to ensure maximum homogeneity of pooled fractions. Lyophilization of these gave the title peptide as a white powder (32 mg).

This material gave one peak emerging at 38 minutes using analytical hplc on a column (0.4 × 25 cm) of Vydac octadecylsilane silica (5 M mesh size, 300 A°) which is pumped at 2 mL/min with a linear gradient of 20 to 40% acetonitrile in 0.1% trifluoracetic acid. Tlc on silica gel using the solvent system 1-butanol: pyridine: acetic acid: water (15:10:3:12) gave one spot of visualized by chlorine-starch spray reagent. Amino acid analysis of a 6 M HCl hydrolysate gave the following amino acid ratios: Asp, 3.08; Thr, 1.08; Ser, 3.01; Glu, 2.22; Gly, 1.10; Ala, 3.30; Val, 0.87; Met, 0.97; Ile, 1.69; Leu, 3.61; Tyr, 1.60; Phe, 1.05; Lys, 2.07; Arg, 3.28.

## Example 4
Preparation of protected N-acetyl human pancreatic growth hormone-releasing factor(1—29)-benzhydrylamine resin

Peptide-benzhydrylamine resin(9—29) (0.98 g, 0.25 mmol) prepared in Example 1 was subjected to the coupling cycles described in Example 2, except that *L*-Tyr was used in place of *D*-Tyr. The 1—29 peptide resin, with the N-terminal Boc group removed, was acetylated by stirring with a 10% solution of acetic anydride/$Et_3N$ in methylene chloride (30 minutes).

## Example 5
Preparation of N-acetyl human pancreatic growth hormone-releasing factor(1—29)-amide (N-acetyl-hpGRF(1—29)-$NH_2$)

The peptide resin (0.75 mmol) described in Example 4 was treated with hydrogen fluoride mixtures and purified as described in Example 3. The lyophilized, purified peptide weighed 16.2 mg.

This material gave one peak emerging at 36.5 minutes using the analytical hplc conditions described in Example 3. Tlc using the conditions also described in Example 3 gave one spot. Amino acid analysis of a 6 M HCl hydrolysate gave the following amino acid ratios: Asp, 3.05; Thr, 1.08; Ser, 2.96; Glu, 2.20; Gly, 1.20; Ala, 3.30; Val, 0.81; Met, 1.00; Ile, 1.65; Leu, 3.50; Tyr, 1.61; Phe, 1.20; Lys, 2.10; Arg, 3.17.

## Example 6

Preparation of protected *D*-alanine$^2$-human pancreatic growth hormone-releasing factor(1—29)-benzhydrylamine resin

Peptide-benzhydrylamine resin (0.98 g, 0.25 mmol) prepared in Example 1 was subjected to the coupling cycles described in Example 2, except that *D*-alanine was used in place of *L*-alanine in position 2 and *L*-tyrosine in place of *D*-tyrosine in position 1.

## Example 7

Preparation of *D*-alanine$^2$-human pancreatic growth hormone-releasing(1—29)-amide [(*D*-Ala$^2$)-hpGRF(1—29)-NH$_2$]

. The peptide resin (0.25 mmol) described in Example 6 is treated with hydrogen fluoride mixtures and purified as described in Example 3. The purified, lyophilized peptide weighed 27.9 mg. This material gave one peak emerging at 35 minutes using the analytical hplc conditions as described in Example 3. Tlc using the conditions described in Example 3 gave one spot. Amino acid analysis of a 6 M HCl hydrolysate gave the following amino acid ratios: Asp, 3.06; Thr, 1.07; Ser, 3.05; Glu, 2.19; Gly, 1.02; Ala, 3.29; Val, 0.94; Met, 1.10; Ile, 1.72; Leu, 3.66; Tyr, 1.62; Phe, 0.90; Lys, 2.07; Arg, 3.19.

## Example 8

Preparation of protected *D*-aspartic acid$^3$-human pancreatic growth hormone-releasing factor(1—29)-benzhydrylamine resin

Peptide-benzhydrylamine resin (0.98 g, 0.25 mmol) prepared in Example 1 was subjected to the coupling cycles described in Example 2, except that *D*-aspartic acid was used in place of *L*-aspartic acid in position 3 and *L*-tyrosine in place of *D*-tyrosine in position 1.

## Example 9

Preparation of *D*-aspartic acid$^3$-human pancreatic growth hormone-releasing(1—29)-amide [(*D*-Asp$^3$)-hpGRF(1—29)-NH$_2$]

The peptide resin (0.25 mmol) described in Example 8 is treated with hydrogen fluoride mixture and purified as described in Example 3. The purified, lyophilized peptide weighed 15.3 mg. This material gave one peak emerging at 34 minutes using the analytical hplc conditions as described in Example 3. Tlc using the condition described in Example 3 gave one spot. Amino acid analysis of a 6 M HCl hydrolysate gave the following amino acid ratios: Asp, 3.17; Thr, 1.22; Ser, 3.08; Glu, 2.36; Gly, 1.06; Ala, 3.30; Val, 0.96; Met, 0.84; Ile, 1.76; Leu, 3.71; Tyr, 1.00; Phe, 0.92; Lys, 2.21; Arg, 3.30.

## Example 10

Preparation of protected N-acetyl-*D*-tyrosine$^1$,*D*-alanine$^2$-human pancreatic growth hormone-releasing factor(1—29)-benzhydrylamine resin

Peptide-benzhydrylamine resin (0.98 g, 0.25 mmol) prepared in Example 1 was subjected to the coupling cycles described in Example 2, except that *D*-alanine was used in place of *L*-alanine in position 2 and *D*-tyrosine in place of *L*-tyrosine in position 1.

## Example 11

Preparation of N-acetyl-*D*-tyrosine$^1$,*D*-alanine$^2$-human pancreatic growth hormone-releasing(1—29)-amide [(N-acetyl-*D*-Tyr$^1$,*D*-Ala$^2$)-hpGRF(1—29)-NH$_2$]

The peptide resin (0.25 mmol) described in Example 10 is treated with hydrogen fluoride mixtures and purified as described in Example 3. The purified, lyophilized peptide weighed 5 mg. This material gave one peak emerging at 33 minutes using the analytical hplc conditions as described in Example 3. Tlc using the conditions described in Example 3 gave one spot. Amino acid analysis of a 6 M HCl hydrolysate gave the following amino acid ratios: Asp, 3.08; Thr, 1.08; Ser, 3.01; Glu, 2.22; Gly, 1.10; Ala, 3.30; Val, 0.87; Met, 0.97; Ile, 1.69; Leu, 3.61; Tyr, 1.60; Phe, 1.05; Lys, 2.07; Arg, 3.28.

## Example 12

Preparation of protected N-acetyl-*D*-tyrosine$^1$,*D*-alanine$^2$,*D*-aspartic acid$^3$-human pancreatic growth hormone-releasing factor(1—29)-benzhydrylamine resin

Peptide-benzhydrylamine resin (0.98 g, 0.25 mmol) prepared in Example 1 was subjected to the coupling cycles described in Example 4, exept that *D*-aspartic acid was used in place of *L*-aspartic acid in position 3, *D*-alanine in place of *L*-aspartic acid in position 2, and *D*-tyrosine in place of *L*-tyrosine in position 1.

Example 13

Preparation of N-acetyl-$D$-tyrosine$^1$,$D$-alanine$^2$,$D$-aspartic acid$^3$-human pancreatic growth hormone-releasing(1—29)-amide [(N-acetyl-$D$Tyr$^1$,$D$-Ala$^2$,$D$-Asp$^3$)-hpGRF(1—29)-NH$_2$]

The peptide resin (0.25 mmol) described in Example 12 is treated with hydrogen fluoride mixtures and purified as described in Example 3. The purified, lyophilized peptide weighed 12 mg. This material gave one peak emerging at 34 minutes using the analytical hplc conditions as described in Example 3. Tlc using the conditions described in Example 3 gave one spot. Amino acid analysis of a 6 M HCl hydrolysate gave the following amino acid ratios: Asp, 3.06; Thr, 1.07; Ser, 3.00; Glu, 2.18; Gly, 1.11; Ala, 3.30; Val, 0.82; Met, 0.97; Ile, 1.62; Leu, 3.43; Tyr, 1.69; Phe, 0.92; Lys, 2.03; Arg, 3.13.

Example 14

Preparation of protected N-acetyl-tosyl-$L$-histidine-1,$D$-alanine-2-human pancreatic growth hormone-releasing factor(1—29)-benzhydrylamine resin

Peptide-benzhydrylamine resin (0.98 g, 0.25 mmol) prepared in Example 1 was subjected to the coupling cycles described in Example 4, except that $D$-alanine in place of $L$-alanine in position 2, and L-tosyl-histidine in place of $L$-tyrosine in position 1.

Example 15

Preparation of N-acetyl-$L$-histidine-1,$D$-alanine$^2$-human pancreatic growth hormone-releasing(1—29) amide [(N-acetyl-His$^1$,$D$-Ala$^2$)-hpGRF(1—29)-NH$_2$]

The peptide resin (0.25 mmol) described in Example 12 is treated with hydrogen fluoride mixtures and purified as described in Example 3. The purified, lyophilized peptide weighed 18 mg. This material gave one peak emerging at 31 minutes using the analytical hplc conditions as described in Example 3. Tlc using the conditions described in Example 3 gave one spot. Amino acid analysis of a 6 M HCl hydrolysate gave the following amino acid ratios: Asp, 3.06; Thr, 1.01; Ser, 3.00; Glu, 2.26; Gly, 1.10; Ala, 3.30; Val, 0.79; Met, 0.97; Ille, 1.69; Leu, 3.71; Tyr, 0.81; Phe, 0.96; His, 0.95; Lys, 2.15; Arg, 3.30.

Example 16

The following N-acyl (R$^1$) growth hormone-releasing factors(1—29) of structural Formula (I) are prepared by using the methods described in Example 4 and Example 5 by substituting the following acid anhydrides for acetic anhydride and the appropriate GRF(1—29), wherein R$^2$ is NH$_2$.

| Anhydride | $\underline{R}^1$ | GRF(1-29) |
|---|---|---|
| HCO-O-COCH$_3$ | HCO | hp |
| (CH$_3$CH$_2$CO)$_2$O | CH$_3$CH$_2$CO | hp |
| [(CH$_3$)$_2$CH-CO]$_2$O | (CH$_3$)$_2$CHCO | hp |
| [CH$_3$(CH$_2$)$_4$CO]$_2$) | CH$_3$(CH$_2$)$_4$CO | hp |
| [(CH$_3$)$_3$CCO]$_2$O | (CH$_3$)$_3$CCO | [$\underline{D}$-Ala$^2$]-hp |
| [(CH$_3$)$_2$CH-CO]$_2$O | (CH$_3$)$_2$CHCO | [$\underline{D}$-Ala$^2$]-hp |
| (CH$_3$(CH$_2$)$_3$CO)$_2$O | CH$_3$(CH$_2$)$_3$CO | [$\underline{D}$-Ala$^2$]-hp |
| (CH$_3$CO)$_2$O | CH$_3$CO | [$\underline{D}$-Ala$^2$]-hp |
| [CH$_3$(CH$_2$)$_4$CO]$_2$O | CH$_3$(CH$_2$)$_4$CO | [$\underline{D}$-Ala$^2$]-hp |
| HCO-O-COCH$_3$ | HCO | [$\underline{D}$-Ala$^2$]-hp |
| CH$_3$(CH$_2$)$_2$CO | CH$_3$(CH$_2$)$_2$CO | [$\underline{D}$-Ala$^2$]-hp |
| (CH$_3$CO)$_2$O | CH$_3$CO | [$\underline{D}$-Asp$^3$]-hp |
| [CH$_3$(CH$_2$)$_4$CO]$_2$CO | CH$_3$(CH$_2$)$_4$CO | [$\underline{D}$-Asp$^3$]-hp |
| (CH$_3$CH$_2$CO)$_2$O | CH$_3$CH$_2$CO | [His$^1$,$\underline{D}$-Ala$^2$]-hp |
| [(CH$_3$)$_2$CHCO]$_2$O | (CH$_3$)$_2$CHCO | [His$^1$,$\underline{D}$-Ala$^2$]-hp |
| CH$_3$(CH$_2$)$_4$CO | CH$_3$(CH$_2$)$_4$CO | [His$^1$,$\underline{D}$-Ala$^2$]-hp |
| HCO-OCOCH$_3$ | HCO | [$\underline{D}$-Tyr$^1$,$\underline{D}$-Ala$^2$]-hp |
| (CH$_3$CH$_2$CO)$_2$O | CH$_3$CH$_2$CO | [$\underline{D}$-Tyr$^1$,$\underline{D}$-Ala$^2$]-hp |
| [(CH$_3$)$_2$CHCO]$_2$O | (CH$_3$)$_2$CHCO | [$\underline{D}$-Tyr$^1$,$\underline{D}$-Ala$^2$]-hp |
| [CH$_3$(CH$_2$)$_3$CO]$_2$O | CH$_3$(CH$_2$)$_3$CO | [$\underline{D}$-Tyr$^1$,$\underline{D}$-Ala$^2$]-hp |
| [CH$_3$(CH$_2$)$_4$CO]$_2$O | CH$_3$(CH$_2$)$_4$CO | [$\underline{D}$-Tyr$^1$,$\underline{D}$-Ala$^2$]-hp |
| [(CH$_3$)$_3$CCO]$_2$O | (CH$_3$)$_3$CCO | [$\underline{D}$-Tyr$^1$,$\underline{D}$-Ala$^2$]-hp |
| HCOOCH$_3$ | HCO | [$\underline{D}$-Tyr$^1$,$\underline{D}$-Ala$^2$,$\underline{D}$-Asp$^3$]-hp |
| (CH$_3$CH$_2$CO)$_2$O | CH$_3$CH$_3$CO | [$\underline{D}$-Tyr$^1$,$\underline{D}$-Ala$^2$,$\underline{D}$-Asp$^3$]-hp |
| [(CH$_3$)$_2$CHCO]$_2$O | (CH$_3$)$_2$CHCO | [$\underline{D}$-Tyr$^1$,$\underline{D}$-Ala$^2$,$\underline{D}$-Asp$^3$]-hp |
| [CH$_3$(CH$_2$)$_3$CO]$_2$O | CH$_3$(CH$_2$)$_3$CO | [$\underline{D}$-Tyr$^1$,$\underline{D}$-Ala$^2$,$\underline{D}$-Asp$^3$]-hp |
| [(CH$_3$)$_3$CCO]$_2$O | (CH$_3$)$_3$CCO | [$\underline{D}$-Tyr$^1$,$\underline{D}$-Ala$^2$,$\underline{D}$-Asp$^3$]-hp |

The superscripts used in this Example, which are assigned to given amino acid residues, indicate the locations of said residues in the amino acid sequence o the synthesized growth hormone-releasing peptides.

## Example 17

Evaluation of peptide effects on growth hormone release in mammals using the rat as the test species

In this evaluation, the procedures described by W. A. Murphy *et al.*, Endocrinology *109*:491—495 (1980), were employed.

In growth hormone (GH) experiments, male rats (Charles Rivers) were anesthetized with NEMBUTAL® (6 mg per 100 g body weight) which also served to maintain stimulated plasma GH levels. Exactly 30 minutes after the rats were anesthetized, 0.5 mL of saline or the test peptide in saline was administered as a SC bolus. A 1 mL blood sample was drawn from the jugular vein 15 minutes after the invention of the peptide in saline. GH levels were determined using NIADDKD rat GH RIA components.

### hpGRF(1-29)-NH$_2$ Structure-Activity Studies

| Analog | Dose (ug/100 g BW) | Plasma GH (ng/mL) |
|---|---|---|
| Saline | - | 333 ± 50 (5)* |
| [N-Ac-D-Tyr$^1$,D-Ala$^2$]-hpGRF(1-29)-NH$_2$ | 0.05 | 499 ± 50 (5) |
| [N-Ac-D-Tyr$^1$,D-Ala$^2$]-hpGRF(1-29)-NH$_2$ | 0.2 | 680 ± 100 (7) |
| [N-Ac-D-Tyr$^1$,D-Ala$^2$]-hpGRF(1-29)-NH$_2$ | 0.8 | 2063 ± 251 (6) |
| [N-Ac-D-Tyr$^1$,D-Ala$^2$]-hpGRF(1-29)-NH$_2$ | 3.2 | 3818 ± 412 (6) |

* Number of rats in parenthesis

| GRF(1-29)-NH$_2$ Analog | Dose (ug/100g BW) | Plasma GH* (ng/ml) |
|---|---|---|
| Saline | – | 366 ± 81 (6) |
| (a) D-Asn$^8$ | 4 | 1296 ± 118 (5) |
| " | 10 | 2397 ± 246 (6) |
| Saline | – | 278 ± 37 (6) |
| (b) D-Ala$^2$-Nle$^{27}$ | 0.1 | 465 ± 75 (6) |
| " | 1.0 | 2287 ± 265 (6) |
| Saline | – | 214 ± 29 (6) |
| (c) His$^1$-D-Ala$^2$-Nle$^{27}$ | 0.1 | 904 ± 111 (6) |
| " | 1.0 | 3379 ± 389 (6) |
| Saline | – | 203 ± 39 (8) |
| (d) D-Ala$^2$-D-Asn$^8$-Nle$^{27}$ | 0.05 | 407 ± 53 (6) |
| " | 0.5 | 1613 ± 138 (6) |
| Saline | – | 383 ± 79 (6) |
| (e) D-Asp$^3$-D-Asn$^8$-Nle$^{27}$ | 0.1 | 810 ± 86 (6) |
| " | 1.0 | 3213 ± 590 (6) |
| (f) D-Ala$^2$-D-Asp$^3$-D-Asn$^8$-Nle$^{27}$ | 0.1 | 767 ± 146 (6) |
| " | 1.0 | 2419 ± 252 (6) |

Potencies were determined by comparison to pooled standards:

| | | |
|---|---|---|
| GRF(1-29)-NH$_2$ | 10 | 1185 ± 74 (78) |
| " | 25 | 2570 ± 121 (70) |

*Values are the mean ± SEM (n).

Example 18

Preparation of *D*-asparagine[8]-human pancreatic growth hormone-releasing(1—29)-amide [(*D*-Asn[8])-hpGRF(1—29)-NH$_2$]

The peptide-benzhydrylamine resin (0.25 mmole of resin) prepared in Example 1 was subjected to the coupling cycles described in Example 2, except that *D*-asparagine was used in place of *L*-asparagine in position 8. The yield was 38 mg, and the peptide had HPLC elution time of 28 minutes at a flow rate of 1.5 mL/minutes. The amino acid analysis gave: Asp, 3.05; Thr, 0.95; Ser, 2.96; Glu, 2.21; Gly, 1.04; Ala, 3.02; Val, 0.95; Ile, 1.85; Leu 2.00; Nle 0.97; Tyr, 2.10; Phe, 0.96; Lys, 1.97; Arg, 3.20.

Example 19

Preparation of *D*-alanine[2], norleucine[27]-human pancreatic growth hormone-releasing(1—29)-amide [(*D*-Ala[2], Nle[27])-hpGRF(1—29)-NH$_2$]

The peptide-benzhydrylamine resin was prepared as in Example 1 with *L*-norleucine replacing *L*-methionine in position 27 of the coupling cycle. A Boc protecting group on *L*-norleucine was used. This material was then coupled in the manner described in Example 2 with *D*-alanine relacing *L*-alanine in position 2. The yield was 71 mg from 0.25 mmole of resin used, and the HPLC elution time was 28.5 minutes with a flow-rate of 1.5 mL/minutes. The amino acid analysis gave: Asp, 3.06; Thr, 1.00; Ser, 2.96; Glu, 2.20; Gly, 1.06; Ala, 3.10; Val, 0.90; Ile, 1.78; Leu 4.00; Nle 1.00; Tyr, 1.96; Phe, 0.88; Lys, 2.00; Arg, 3.14.

Example 20

Preparation of histidine[1], *D*-alanine[2], norleucine[27]-human pancreatic growth hormone-releasing(1—29)-amide [(His[1], *D*-Ala[2], Nle[27])-hpGRF(1—29)-NH$_2$]

The peptide-benzhydrylamine resin was prepared as in Example 1 with *L*-norleucine replacing *L*-methionine in position 27 of the coupling cycle. This material was then coupled in the manner described in Example 2 with *L*-histidine replacing *L*-tyrosine in position 1 and *D*-alanine replacing *L*-alanine in position 2. The yield was 23 mg from 0.25 mmole of resin used, and the HPLC elution time was 27.5 minutes with a flow-rate of 1.5 mL/minutes. The amino acid analysis gave: Asp, 3.13; Thr, 1.00; Ser, 2.98; Glu, 2.27; Gly, 1.10; Ala, 3.15; Val, 0.99; Ile, 1.88; Leu 4.20; Nle 1.05; Tyr, 0.98; His, 1.17; Lys, 2.01; Arg, 3.11.

Example 21

Preparation of *D*-alanine[2], *D*-asparagine[8], norleucine[27]-human pancreatic growth hormone-releasing(1—29)-amide [(*D*-Ala[2], *D*-Asn[8], Nle[27])-hpGRF(1—29)-NH$_2$]

The peptide-benzhydryl resin was prepared as in Example 1 with *L*-norleucine replacing *L*-methionine in position 27 of the coupling cycle. This material was then coupled in the manner described in Example 2 with *D*-alanine replacing *L*-alanine in position 2 and *D*-asparagine replacing *L*-asparagine in position 8. The yield was 41 mg from 0.25 mmole of resin used, and the HPLC elution time was 28.5 minutes at a flow-rate of 1.5 mL/minutes. The amino acid analysis gave: Asp, 2.99; Thr, 1.00; Ser, 2.87; Glu, 2.14; Gly, 1.06; Ala, 3.04; Val, 0.93; Ile, 1.80; Leu 4.02; Nle 0.98; Tyr, 1.99; Phe, 0.90; Lys, 2.01; Arg, 3.08.

Example 22

Preparation of *D*-aspartic acid[3], *D*-asparagine[8], norleucine[27]-human pancreatic growth hormone-releasing(1—29)-amide [(*D*-Asp[3], *D*-Asn[8], Nle[27])-hpGRF(1—29)-NH$_2$]

The peptide-benzhydryl resin was prepared as in Example 1 with *L*-norleucine replacing *L*-methionine in position 27 of the coupling cycle. This material was then coupled in the manner described in Example 2 with *D*-aspartic acid replacing *L*-aspartic acid in position 3 and *D*-asparagine replacing *L*-asparagine in position 8. The yield was 114 mg starting with 0.25 mmole of resin, and the HPLC elution time was 29.1 minutes at a flow-rate of 1.5 mL/minutes. The amino acid analysis gave: Asp, 2.99; Thr, 1.00; Ser, 2.85; Glu, 2.16; Gly, 1.03; Ala, 3.03; Val, 0.94; Ile, 1.82; Leu 4.03; Nle 0.99; Tyr, 1.97; Phe, 0.92; Lys, 2.06; Arg, 3.05.

Example 23

Preparation of *D*-alanine[2], *D*-aspartic acid[3], *D*-asparagine[8], norleucine[27]-human pancreatic growth hormone-releasing(1—29)-amide [(*D*-Ala[2], *D*-Asp[3], *D*-Asn[8], Nle[27])-hpGRF(1—29)-NH$_2$]

The peptide-benzhydryl resin was prepared as in Example 1 with *L*-norleucine replacing *L*-methionine in position 27 of the coupling cycle. This material was then coupled in the manner described in Example 2 with *D*-alanine replacing *L*-alanine in position 2, *D*-aspartic acid replacing *L*-aspartic acid in position 3 and *D*-asparagine replacing *L*-asparagine in position 8. The yield was 67 mg starting with 0.25 mmole of resin, and the HPLC elution time was 28 minutes at a flow-rate of 1.5 mL/minutes. The amino acid analysis gave: Asp, 3.01; Thr, 1.00; Ser, 2.90; Glu, 2.20; Gly, 1.05; Ala, 3.08; Val, 0.95; Ile, 1.86; Leu 4.01; Nle 0.96; Tyr, 1.95; Phe, 0.93; Lys, 2.05; Arg, 3.11.

**Claims for the Contracting States: BE CH DE FR GB IT LI LU NL SE**

1. Peptide characterized by the formula:

```
R1-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-R2;
```

$$(I)$$

or

```
R1-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-Gln-Gln-Gly-Glu-Ser-Asn-Gln-Glu-Arg-Gly-Ala-R2
```

$$(II)$$

or

```
R1-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-Gln-Gln-Gly-Glu-Ser-Asn-Glu-Glu-Arg-Gly-Ala-Arg-
Ala-Arg-Leu-R2;
```

$$(III)$$

wherein $R^1$ is hydrogen or $C_1$—$C_6$ straight- or branched-chain alkanoyl; $R^2$ is $NR^3R^4$ or $OR^3$; $R^3$ and $R^4$ are selected from the group consisting of hydrogen and a straight- or branched-chain alkyl group containing one to six carbon atoms; A is tyrosyl, D-tyrosyl, or histidyl, B is an alanyl, D-alanyl, C is aspartyl or D-aspartyl; X is asparaginyl or D-asparaginyl; Y is norleucyl or methionyl; with the proviso that either B and/or C and/or X be a D amino acid; and the pharmaceutically acceptable salts thereof.

2. A peptide according to Claim 1, wherein $R^1$ is selected from hydrogen or $C_1$—$C_3$ alkanoyl; $R^2$ is $NR^3R^4$ or $OR^3$; $R^3$ and $R^4$ are each selected from hydrogen and $C_1$—$C_3$ alkyl; A is tyrosyl, D-tyrosyl, histidyl; B is alanyl or D-alanyl; C is aspartyl or D-aspartyl; X is asparaginyl or D-asparaginyl; Y is norleucyl or methionyl; with the above-mentioned proviso; and the pharmaceutically acceptable salts thereof.

3. A peptide according to Claim 1, having the Formula (I) structure wherein $R^1$ is selected from hydrogen or acetyl; $R^2$ is $NH_2$; A is tyrosyl, D-tyrosyl, or histidyl; B is alanyl or D-alanyl; C is aspartyl or D-aspartyl; X is asparaginyl or D-asparaginyl; Y is norleucyl or methionyl; with the above-mentioned proviso; and the pharmaceutically acceptable salts thereof.

4. The peptide according to Claim 1 characterized by the formula:

4. The peptide according to Claim 1 characterized by the formula:

Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts;

Tyr-Ala-*D*-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts;

Ac-His-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts;

Tyr-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts;

Tyr-*D*-Als-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ and its pharmaceutically acceptable salts;

His-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ and its pharmaceutically acceptable salts;

Tyr-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ and its pharmaceutically acceptable salts;

Tyr-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ and its pharmaceutically acceptable salts;

Tyr-*D*-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ and its pharmaceutically acceptable salts; or

Ac-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ and its pharmaceutically acceptable salts.

5. A method for increasing the release of growth hormone in non human mammals, said method characterized by administering thereto from 0.000001 to 0.1 mg/kg of mammalian body weight/day of a peptide having the formula:

$$R^1\text{-A-B-C-Ala-Ile-Phe-Thr-X-Ser-Tyr-Arg-Lys-Val-Leu-}$$
$$\text{Gly-Gln-Leu-Ser-Ala-Arg-Lys-Leu-Leu-Gln-Asp-Ile-Y-Ser-}$$
$$\text{Arg-}R^2;$$

(I)

or

$$R^1\text{-A-B-C-Ala-Ile-Phe-Thr-X-Ser-Tyr-Arg-Lys-Val-Leu-}$$
$$\text{Gly-Gln-Leu-Ser-Ala-Arg-Lys-Leu-Leu-Gln-Asp-Ile-Y-Ser-}$$
$$\text{Arg-Gln-Gln-Gly-Glu-Ser-Asn-Gln-Glu-Arg-Gly-Ala-}R^2$$

(II)

or

$$R^1\text{-A-B-C-Ala-Ile-Phe-Thr-X-Ser-Tyr-Arg-Lys-Val-Leu-}$$
$$\text{Gly-Gln-Leu-Ser-Ala-Arg-Lys-Leu-Leu-Gln-Asp-Ile-Y-Ser-}$$
$$\text{Arg-Gln-Gln-Gly-Glu-Ser-Asn-Glu-Glu-Arg-Gly-Ala-Arg-}$$
$$\text{Ala-Arg-Leu-}R^2;$$

(III)

wherein $R^1$, $R^2$, A, B, C, X and Y are as defined in Claim 1, and with the same proviso set out in claim 1.

6. A method according to Claim 5, wherein $R^1$ is selected from hydrogen or $C_1$—$C_3$ alkanoyl; $R^2$ is $NR^3R^4$ or $OR^3$; $R^3$ and $R^4$ are each selected from hydrogen and $C_1$—$C_3$ alkyl; A is tyrosyl, *D*-tyrosyl, or histidyl; B is alanyl or *D*-alanyl; C is aspartyl or *D*-aspartyl; X is asparaginyl or *D*-asparaginyl; Y is norleucyl or methionyl; with the above-mentioned proviso; and the pharmaceutically acceptable salts thereof.

21

7. A method according to Claim 5, wherein the peptide is of the Formula (I); $R^1$ is hydrogen or acetyl; $R^2$ is $NH_2$; A is tyrosyl, *D*-tyrosyl, or histidyl; B is alanyl or *D*-alanyl; C is aspartyl or *D*-aspartyl; X is asparaginyl or *D*-asparaginyl; Y is norleucyl or methionyl; with the above-mentioned proviso; and the pharmaceutically acceptable salts thereof.

8. A method according to Claim 5, wherein the peptide is

[D-Ala²]-hpGRF(1-29)-NH₂; Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ and its pharmaceutically acceptable salts; Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ and its pharmaceutically acceptable salts; His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ and its pharmaceutically acceptable salts; Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ and its pharmaceutically acceptable salts; Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ and its pharmaceutically acceptable salts; Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ and its pharmaceutically acceptable salts; or Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ and its pharmaceutically acceptable salts.

9. A process for the preparation of a peptide having the formula,

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

$$(I)$$

or

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

$$(II)$$

or

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

$$(III)$$

wherein $R^1$, $R^2$, A, B, C, X and y are defined as in Claim 1, with the same proviso of claim 1, and the pharmaceutically acceptable salts thereof; said method comprising: attaching a C-terminal protected amino acid to a resin in the present of a coupling agent; washing with an inert solvent; deprotecting in the presence of an acid; repeating this sequence with the subsequent protected amino acids as defined in formula (I), (II) and (III), stepwise, from the C-terminus of the peptide; and finally detaching the peptide from the resin in the presence of an acid to afford the peptide of formula (I), (II) and (III), wherein said resin is BHA or p-ME—BHA; or detaching the peptide from the resin with ammonia, alkylamine or dialkylamine and deptrotecting with an acid to afford the peptide of formula (I), (II) and (III), wherein said resin is a chlormethylated or hydroxymethylated resin; or detaching the peptide from the resin in the presence of an acid to afford the peptide of the formula (I), (II) or (III), wherein said resin is a chlormethylated or hydroxymethylated resin; or detaching the peptide from the resin in the presence of a base and an $R^3OH$ alcohol, followed by acid treatment to afford the peptide of the formula (I), (II) or (III), wherein $R^3$ is a straight- or branched chain alkyl group containing one to six carbon atoms and said resin is a chloromethylated or hydroxymethylated resin.

10. A process for the preparation of a peptide according to claim 9, wherein said peptide is characterized by the formula:

23

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Tyr-Ala-D-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Ac-His-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Tyr-D-Als-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts; or

Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts.

11. A peptide characterized by the formula:

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts or

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts.

12. A process for the preparation of a peptide having

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts or

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts.

said method comprising: attaching a C-terminal protected amino acid to a resin in the present of a coupling agent; washing with an inert solvent; deprotecting in the presence of an acid; repeating this sequence with the subsequent protected amino acids as defined in the above peptide formulae, stepwise, from the C-terminus of the peptide; and finally detaching the peptide from the resin in the presence of an acid to afford the peptides of the above formula, wherein said resin is BHA or p-ME—BHA; or detaching the peptide from the resin with ammonia, alkylamine or dialkylamine and deprotecting with an acid to afford the peptides of the above formulae, wherein said resin is a chlormethylated or hydroxymethylated resin; or detaching the peptide from the resin in the presence of an acid to afford the peptide of the above formulae, wherein said resin is a chloromethylated or hydroxymethylated resin; or detaching the peptide from the resin in the presence of a base and an R$^3$OH alcohol, followed by acid treatment to afford the peptide of the above formulae, wherein R$^3$ is a straight- or branched chain alkyl group containing one to six carbon atoms and said resin is a chloromethylated or hydroxymethylated resin.

24

**Claims for the Contracting State: AT**

1. A process for the preparation of a Peptide having the formula:

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

(I)

or

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

(II)

or

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

(III)

wherein $R^1$ is hydrogen or $C_1$—$C_6$ straight- or branched-chain alkanoyl; $R^2$ is $NR^3R^4$ or $OR^3$; $R^3$ and $R^4$ are selected from the group consisting of hydrogen and a straight- or branched-chain alkyl group containing one to six carbon atoms; A is tyrosyl, D-tyrosyl, or histidyl, B is an alanyl, or D-alanyl, C is aspartyl or D-aspartyl; X is asparaginyl or D-asparaginyl; Y is norleucyl or methionyl; with the proviso that either B and/or C and/or X be a D amino acid; and the pharmaceutically acceptable salts thereof; said method comprising: attaching a C-terminal protected amino acid to a resin in the presence of a coupling agent; washing with an inert solvent; deprotecting in the presence of an acid; repeating this sequence with the subsequent protected amino acids as defined in formula (I), (II) and (III), stepwise, from the C-terminus of the peptide; and finally detaching the peptide from the resin in the presence of an acid to afford the peptide of formula (I), (II) and (III), wherein said resin is BHA or p-ME—BHA; or detaching the peptide from the resin with ammonia, alkylamine or dialkylamine and deprotecting with an acid to afford the peptide of formula (I), (II) and (III), wherein said resin is a chlormethylated or hydroxymethylated resin; or detaching the peptide from the resin in the presence of an acid to afford the peptide of the formula (I), (II) or (III), wherein said resin is a chlormethylated or hydroxymethylated resin; or detaching the peptide from the resin in the presence of a base and an $R^3OH$ alcohol, followed by acid treatment to afford the peptide of the formula (I), (II) or (III), wherein $R^3$ is a straight- or branched chain alkyl group containing one to six carbon atoms and said resin is a chloromethylated or hydroxymethylated resin.

2. A process for the preparation of a peptide according to Claim 1, wherein said peptide is characterized by the formula:

Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Tyr-Ala-*D*-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Ac-His-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Tyr-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts;

Tyr-*D*-Als-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

His-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

Tyr-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

Tyr-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts;

Tyr-*D*-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ and its pharmaceutically acceptable salts; or

Ac-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts.

3. A process for the preparation of a peptide having

*D*-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts or

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ and its pharmaceutically acceptable salts.

said method comprising: attaching a C-terminal protected amino acid to a resin in the presence of a coupling agent; washing with an inert solvent; deprotecting in the presence of an acid; repeating this sequence with the subsequent protected amino acids as defined in the above peptide formulae, stepwise, from the C-terminus of the peptide; and finally detaching the peptide from the resin in the presence of an acid to afford the peptides of the above formula, wherein said resin is BHA or *p*-ME—BHA; or detaching the peptide from the resin with ammonia, alkylamine or dialkylamine and deprotecting with an acid to afford the peptides of the above formulae, wherein said resin is a chlormethylated or hydroxymethylated resin; or detaching the peptide from the resin in the presence of an acid to afford the peptide of the above formulae, wherein said resin is a chloromethylated or hydroxymethylated resin; or detaching the peptide from the resin in the presence of a base and an R$^3$OH alcohol, followed by acid treatment to afford the peptide of the above formulae, wherein R$^3$ is a straight- or branched chain alkyl group containing one to six carbon atoms and said resin is a chloromethylated or hydroxymethylated resin.

**Patentansprüche für die Vertragsstaaten: BE CH DE FR GB IT LI LU NL SE**

1. Peptid, gekennziechnet durch die Formel:

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

$$(I)$$

oder

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

$$(II)$$

oder

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

$$(III)$$

wobei $R^1$ für Wasserstoff oder $C_1$—$C_6$-geradkettiges oder verzweigtkettiges Alkanoyl steht; $R^2$ für $NR^3R^4$ oder $OR^3$ steht; $R^3$ und $R^4$ ausgewählt sind aus der Gruppe bestehend aus Wasserstoff und einer geradkettigen oder verzweigtkettigen Alkylgruppe enthaltend 1 bis 6 Kohlenstoffatome; A für Tyrosyl, *D*-Tyrosyl oder Histidyl steht; B für Alanyl oder *D*-Alanyl steht; C für Aspartyl oder *D*-Aspartyl steht; X für Asparaginyl oder *D*-Asparaginyl steht; Y für Norleucyl oder Methionyl steht; mit der Maßgabe, daß entweder B und/oder C und/oder X eine D-Aminosäure ist; sowie die pharmazeutisch akzeptablen Salze derselben.

2. Peptide gemäß Anspruch 1, wobei $R^1$ ausgewählt ist aus Wasserstoff oder $C_1$—$C_3$-Alkanoyl; $R^2$ für $NR^3R^4$ oder $OR^3$ steht; $R^3$ und $R^4$ jeweils ausgewählt sind aus Wasserstoff und $C_1$—$C_3$-Alkyl; A für Tyrosyl, *D*-Tyrosyl oder Histidyl steht; B für Alanyl oder *D*-Alanyl steht; C für Aspartyl oder *D*-Aspartyl steht; X für Asparaginyl oder *D*-Asparaginyl steht; Y für Norleucyl oder Methionyl steht; mit der oben erwähnten Maßgabe; sowie die pharmazeutisch akzeptablen Salze derselben.

3. Peptid gemäß Anspruch 1, mit der Struktur der Formel I, wobei $R^1$ ausgewählt ist aus Wasserstoff oder Acetyl; $R^2$ für $NH_2$ steht; A für Tyrosyl, *D*-Tyrosyl oder Histidyl steht; B für Alanyl oder *D*-Alanyl steht; C für Aspartyl oder *D*-Aspartyl steht; X für Asparaginyl oder *D*-Asparaginyl steht; Y für Norleucyl oder Methionyl steht; mit der oben erwähnten Maßgabe; sowie die pharmazeutisch akzeptablen Salze derselben.

4. Peptid gemäß Anspruch 1, gekennzeichnet durch die Formel:

Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptablen Salze;

Tyr-Ala-*D*-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptablen Salze;

Ac-His-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptable Salze;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptablen Salze;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptablen Salze;

Tyr-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptablen Salze;

Tyr-*D*-Ala-Asp-Ala-Ile-Phe-Thr-Asn-Ser-Tyr-Arg-Lys-Val-Leu-Gly-Gln-Leu-ser-Ala-Arg-Lys-Leu-Leu-Gln-Asp-Ile-Nle-Ser-Arg-$NH_2$ und dessen pharmazeutisch akzeptablen Salze;

His-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ und dessen pharmazeutisch akzeptablen Salze;

Tyr-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ und dessen pharmazeutisch akzeptablen Salze;

Tyr-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ und dessen pharmazeutisch akzeptablen Salze;

Tyr-*D*-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ und dessen pharmazeutisch akzeptablen Salze; oder

Ac-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ und dessen pharmazeutisch akzeptablen Salze.

5. Methode zur Steigerung der Ausschüttung von Wachstumshormon bei Säugetieren (keine Menschen), wobei die Methode gekennzeichnet ist durch Verabreichung von 0,000001 bis 0,1 mg/kg Säugetier-Körpergewicht pro Tag eines Peptids mit der Formel

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

(I)

oder

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

(II)

oder

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

(III)

wobei $R^1$, $R^2$, A, B, C, X und Y wie in Anspruch 1 definiert sind und mit der gleichen Maßgabe, wie sie in Anspruch 1 angegeben ist.

6. Methode gemäß Anspruch 5, wobei $R^1$ ausgewählt ist aus Wasserstoff oder $C_1$—$C_3$-Alkanoyl; $R^2$ für $NR^3R^4$ oder $OR^3$ steht; $R^3$ und $R^4$ jeweils ausgewählt sind aus Wasserstoff und $C_1$—$C_3$-Alkyl; A für Tyrosyl, *D*-Tyrosyl oder Histidyl steht; B für Alanyl oder *D*-Alanyl steht; C für Aspartyl oder *D*-Aspartyl steht; X für Asparaginyl oder *D*-Asparaginyl steht; Y für Norleucyl oder Methionyl steht; mit der oben erwähnten Maßgabe; sowie die pharmazeutisch akzeptablen Salze derselben.

7. Methode gemäß Anspruch 5, wobei das Peptid die Formel (I) aufweist; R¹ für Wasserstoff oder Acetyl steht; R² für NH₂ steht, A für Tyrosyl, *D*-Tyrosyl oder Histidyl steht; B für Alanyl oder *D*-Alanyl steht; C für Aspartyl oder *D*-Aspartyl steht; X für Asparaginyl oder *D*-Asparaginyl steht; Y für Norleucyl oder Methionyl steht; mit der oben erwähnten Maßgabe; sowie die pharmazeutisch akzeptablen Salze derselben.

8. Methode gemäß Anspruch 5, wobei das Peptid [*D*-Ala²]-hpGRF(1—29) —NH₂ ist:

Tyr-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze;

Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptable Salze;

His-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptable Salze;

Tyr-*D*-Als-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptable Salze;

Tyr-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptable Salze;

Tyr-*D*-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptable Salze; oder

Ac-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze.

9. Verfahren zur Herstellung eines Peptids mit der Formel

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

$$(I)$$

oder

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

$$(II)$$

oder

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

$$(III)$$

wobei R¹, R², A, B, C, X und Y wie in Anspruch 1 definiert sind, mit der gleichen Maßgabe von Anspruch 1; sowie die pharmazeutisch akzeptablen Salze derselben; wobei das Verfahren umfaßt: Anknüpfung einer C-terminal-geschützten Aminosäure an ein Harz in Gegenwart eines Kupplungsmittels; Waschen mit einem inerten Lösungsmittel; Schutzgruppenabspaltung in Gegenwart einer Säure; Wiederholung dieser Sequenz, schrittweise mit den folgenden geschützten Aminosäuren, wie sie in Formel (I), (II) und (III) definiert sind, vom C-Ende des Peptids her und schließlich Ablösung des Peptids von dem Harz in Gegenwart einer Säure, um das Peptid der Formel (I), (II) oder (III) zu erhalten, wobei das Harz BHA *p*-ME—BHA ist; oder Ablösung des Peptids von dem Harz mit Ammoniak, Alkylamin oder Dialkylamin und Schutzgruppenentfernung mit einer Säure, um das Peptid der Formel (I), (II) oder (III) zu erhalten, wobei das Harz ein chlormethyliertes oder hydroxymethyliertes Harz ist; oder Ablösung des Peptids von dem Harz in Gegenwart einer Säure, um das Peptid der Formel (I), (II) oder (III) zu erhalten, wobei das Harz ein chlormethyliertes oder hydroxymethyliertes Harz ist; oder Ablösung des Peptids von dem Harz in Gegenwart einer Base und einem R³OH-Alkohol, gefolgt von Säurebehandlung, um das Peptid der Formel (I), (II) oder (III) zu erhalten, wobei R³ eine geradkettige oder verzweigtkettige Alkylgruppe, enthaltend 1 bis 6 Kohlenstoffatome, ist und das Harz ein chlormethyliertes oder hydroxymethyliertes Harz ist.

10. Verfahren zur Herstellung eines Peptids gemäß Anspruch 9, wobei das Peptid gekennzeichnet ist durch die Formel

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptablen Salze;

Tyr-Ala-D-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptablen Salze;

Ac-His-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptablen Salze;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptablen Salze;

Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptablen Salze;

Tyr-D-Ala-Asp-Ala-Ile-Phe-Thr-Asn-Ser-Tyr-Arg-Lys-Val-Leu-Gly-Gln-leu-Ser-Ala-Arg-Lys-Leu-Leu-Gln-Asp-Ile-Nle-Ser-Arg-NH₂ und dessen pharmazeutisch akzeptablen Salze;

His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptablen Salze;

Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptablen Salze;

Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptablen Salze;

Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ und dessen pharmazeutisch akzeptablen Salze; oder

Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptablen Salze.

11. Peptid, gekennzeichnet durch die Formel

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze oder

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze.

12. Verfahren zur Herstellung eines Peptids mit der Formel

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze oder

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ und dessen pharmazeutisch akzeptable Salze.

wobei das Verfahren umfaßt: Anknüpfen einer C-terminal-geschützten Aminosäure an ein Harz in Gegenwart eines Kupplungsmittels; Waschen mit einem inerten Lösungsmittel; Schutzgruppenabspaltung in Gegenwart einer Säure; Wiederholung dieser Sequenz, schrittweise mit den folgenden geschützten Aminosäuren, wie sie in den obigen Peptidformeln definiert sind, vom C-Terminus des Peptids her; und schließlich Ablösung des Peptids von dem Harz in Gegenwart einer Säure, um die Peptide der obigen Formeln zu erhalten, wobei das Harz BHA oder p-ME—BHA ist; oder Ablösung des Peptids von dem Harz mit Ammoniak, Alkylamin oder Dialkylamin und Schutzgruppenentfernung mit einer Säure, um die Peptide der obigen Formeln zu erhalten, wobei das Harz ein chlormethyliertes oder hydroxymethyliertes Harz ist; oder Ablösen des Peptids von dem Harz in Gegenwart einer Base und einem R³OH-Alkohol, gefolgt von Säurebehandlung, um das Peptid der obigen Formeln zu erhalten, wobei R³ eine geradkettige oder verzweigtkettige Alkylgruppe, enthaltend 1 bis 6 Kohlenstoffatome, ist und das Harz ein chlormethylierts oder hydroxymethyliertes Harz ist.

**Patentansprüche für den Vertragsstaat: AT**

1. Verfahren zur Herstellung eines Peptid mit der Formel:

$$R^1\text{-}A\text{-}B\text{-}C\text{-}Ala\text{-}Ile\text{-}Phe\text{-}Thr\text{-}X\text{-}Ser\text{-}Tyr\text{-}Arg\text{-}Lys\text{-}Val\text{-}Leu\text{-}$$
$$Gly\text{-}Gln\text{-}Leu\text{-}Ser\text{-}Ala\text{-}Arg\text{-}Lys\text{-}Leu\text{-}Leu\text{-}Gln\text{-}Asp\text{-}Ile\text{-}Y\text{-}Ser\text{-}$$
$$Arg\text{-}R^2;$$

$$(I)$$

oder

$$R^1\text{-}A\text{-}B\text{-}C\text{-}Ala\text{-}Ile\text{-}Phe\text{-}Thr\text{-}X\text{-}Ser\text{-}Tyr\text{-}Arg\text{-}Lys\text{-}Val\text{-}Leu\text{-}$$
$$Gly\text{-}Gln\text{-}Leu\text{-}Ser\text{-}Ala\text{-}Arg\text{-}Lys\text{-}Leu\text{-}Leu\text{-}Gln\text{-}Asp\text{-}Ile\text{-}Y\text{-}Ser\text{-}$$
$$Arg\text{-}Gln\text{-}Gln\text{-}Gly\text{-}Glu\text{-}Ser\text{-}Asn\text{-}Gln\text{-}Glu\text{-}Arg\text{-}Gly\text{-}Ala\text{-}R^2$$

$$(II)$$

oder

$$R^1\text{-}A\text{-}B\text{-}C\text{-}Ala\text{-}Ile\text{-}Phe\text{-}Thr\text{-}X\text{-}Ser\text{-}Tyr\text{-}Arg\text{-}Lys\text{-}Val\text{-}Leu\text{-}$$
$$Gly\text{-}Gln\text{-}Leu\text{-}Ser\text{-}Ala\text{-}Arg\text{-}Lys\text{-}Leu\text{-}Leu\text{-}Gln\text{-}Asp\text{-}Ile\text{-}Y\text{-}Ser\text{-}$$
$$Arg\text{-}Gln\text{-}Gln\text{-}Gly\text{-}Glu\text{-}Ser\text{-}Asn\text{-}Glu\text{-}Glu\text{-}Arg\text{-}Gly\text{-}Ala\text{-}Arg\text{-}$$
$$Ala\text{-}Arg\text{-}Leu\text{-}R^2;$$

$$(III)$$

wobei $R^1$ für Wasserstoff oder $C_1$—$C_6$-geradkettiges oder verzweigtkettiges Alkanoyl steht; $R^2$ für $NR^3R^4$ oder $OR^3$ steht; $R^3$ und $R^4$ ausgewählt sind aus der Gruppe bestehend aus Wasserstoff und einer geradkettigen oder verzweigtkettigen Alkylgruppe enthaltend 1 bis 6 Kohlenstoffatome; A für Tyrosyl, *D*-Tyrosyl oder Histidyl steht; B für Alanyl oder *D*-Alanyl steht; C für Aspartyl oder *D*-Aspartyl steht; X für Asparaginyl oder *D*-Asparaginyl steht; Y für Norleucyl oder Methionyl steht; mit der Maßgabe, daß entweder B und/oder C und/oder X eine D-Aminosäure ist; sowie die pharmazeutisch akzeptablen Salze derselben, wobei das Verfahren umfaßt: Anknüpfen einer C-terminal-geschützten Aminosäure an ein Harz in Gegenwart eines Kupplungsmittels; Waschen mit einem inerten Lösungsmittel; Schutzgruppentfernung in Gegenwart einer Säure; Wiederholung dieser Sequenz, schrittweise mit den folgenden geschützten Aminosäuren, wie sie in den obigen Peptidformeln definiert sind, vom C-Terminus des Peptids; und schließlich Ablösen des Peptids von dem Harz in Gegenwart einer Säure, um die Peptid der obigen Formeln zu erhalten, wobei das Harz BHA oder *p*-ME—BHA ist; oder Ablösen des Peptids von dem Harz mit Ammoniak, Alkylamin oder Dialkylamin und Schutzgruppenentfernung mit einer Säure, um die Peptide der obigen Formeln zu erhalten, wobei das Harz ein chlormethyliertes oder hydroxymethyliertes Harz ist; oder Ablösung des Peptids von dem Harz in Gegenwart einer Säure, um das Peptid der obigen Formeln zu erhalten, wobei das Harz ein chlormethyliertes oder hydroxymethyliertes Harz ist; oder Ablösen des Peptids von dem Harz in Gegenwart einer Base und eines $R^3OH$-Alkohols, gefolgt von Säurebehandlung, um das Peptid der obigen Formeln zu erhalten, wobei $R^3$ eine geradkettige oder verzweigtkettige Alkylgruppe, enthaltend 1 bis 6 Kohlenstoffatome, ist und das Harz ein chlormethyliertes oder hydroxymethyliertes Harz ist.

2. Verfahren zur Herstellung eines Peptids gemäß Anspruch 1, wobei das Peptid gekennzeichnet ist durch die Formel

Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptablen Salze;

Tyr-Ala-*D*-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptablen Salze;

Ac-His-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptable Salze;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptablen Salze;

Ac-*D*-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptablen Salze;

Tyr-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptablen Salze;

Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ und dessen pharmazeutisch akzeptablen Salze;

His-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ und dessen pharmazeutisch akzeptablen Salze;

Tyr-*D*-Ala-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ und dessen pharmazeutisch akzeptablen Salze;

Tyr-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ und dessen pharmazeutisch akzeptablen Salze;

Tyr-*D*-Ala-*D*-Asp-Ala-Ile-Phe-Thr-*D*-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ und dessen pharmazeutisch akzeptablen Salze; oder

Ac-Tyr-*D*-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptablen Salze.

3. Verfahren zur Herstellung eines Peptids der Formel

*D*-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptable Salze oder

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ und dessen pharmazeutisch akzeptable Salze.

wobei das Verfahren umfaßt: Anknüpfen einer C-terminal-geschützten Aminosäure an ein Harz in Gegenwart eines Kupplungsmittels; Waschen mit einem inerten Lösungsmittel; Schutzgruppenentfernung in Gegenwart einer Säure; Wiederholung dieser Sequenz, schrittweise mit den folgenden geschützten Aminosäuren, wie sie in den obigen Peptidformeln definiert sind, vom C-Terminus des Peptids; und schließlich Ablösen des Peptids von dem Harz in Gegenwart einer Säure, um die Peptide der obigen Formeln zu erhalten, wobei das Harz BHA oder *p*-ME—BHA ist; oder Ablösen des Peptids von dem Harz mit Ammoniak, Alkylamin oder Dialkylamin und Schutzgruppenentfernung mit einer Säure, um die Peptide der obigen Formeln zu erhalten, wobei das Harz ein chlormethyliertes oder hydroxymethyliertes Harz ist; oder Ablösen des Peptids von dem Harz in Gegenwart einer Säure, um das Peptid der obgien Formeln zu erhalten, wobei das Harz ein chlormethyliertes oder hydroxymethyliertes Harz ist; oder Ablösen des Peptids von dem Harz in Gegenwart einer Base und eines R$^3$OH-Alkohols, gefolgt von Säurebehandlung, um das Peptid der obigen Formeln zu erhalten, wobei R$^3$ eine geradkettige oder verzweigtkettige Alkylgruppe, enthaltend 1 bis 6 Kohlenstoffatome, ist und das Harz ein chlormethyliertes oder hydroxymethyliertes Harz ist.

**Revendications pour les Etats contractants: BE CH DE FR GB IT LI LU NL SE**

1. Peptide caractérisé par la formule:

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

(I)

ou

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

(II)

ou

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

(III)

dans laquelle $R^1$ est un atome d'hydrogène ou un groupe alcanoyle en $C_1$—$C_6$ à chaîne droite ou ramifiée; $R^2$ est $NR^3R^4$ ou $OR^3$; $R^3$ et $R^4$ sont choisis dans le groupe constitué par un atome d'hydrogène et un groupe alkyle à chaîne droite ou ramifiée contenant un à six atomes de carbone; A est un groupe tyrosyle, D-tyrosyle ou histidyle; B est un groupe alanyle ou D-alanyle; C est un groupe aspartyle ou D-aspartyle; X est un groupe asparaginyle ou D-asparaginyle; Y est un groupe norleucyle ou méthionyle; sous réserve que B et/ou C et/ou X soient un D-amino-acide; et ses sels pharmaceutiquement acceptables.

2. Peptide selon la revendication 1, où $R^1$ est choisi parmi un atome d'hydrogène ou un groupe alcanoyle en $C_1$—$C_3$; $R^2$ est $NR^3R^4$ ou $OR^3$; $R^3$ et $R^4$ sont choisis chacun parmi un atome d'hydrogène et un groupe alkyle en $C_1$—$C_3$; A est un groupe tyrosyle, D-tyrosyle ou histidyle; B est un groupe alanyle ou D-alanyle; C est un groupe aspartyle ou D-aspartyle; X est un groupe asparaginyle ou D-asparaginyle; Y est un groupe norleucyle ou méthionyle; avec la réserve précitée; et ses sels pharmaceutiquement acceptables.

3. Peptide selon la revendication 1 ayant la structure de formule (I) dans laquelle $R^1$ est choisi parmi un atome d'hydrogène ou un groupe acétyle; $R^2$ est $NH_2$; A est un groupe tyrosyle, D-tyrosyle ou histidyle; B est un groupe alanyle ou D-alanyle; C est un groupe aspartyle ou D-aspartyle; X est un groupe asparaginyle ou D-asparaginyle; Y est un groupe norleucyle ou méthionyle; avec la réserve précitée; et ses sels pharmaceutiquement acceptables.

4. Peptide selon la revendication 1 caractérisé par la formule:

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;

Tyr-Ala-D-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;

Ac-His-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;

Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;

His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;

Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;

Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;

Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables; ou

Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables.

5. Procédé pour accroître la libération de l'hormone de croissance chez des mammifères non humains, caractérisé par l'administration à ceux-ci de 0,000001 à 0,1 mg/kg de poids corporel du mammifère/jour d'un peptide de formule:

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

(I)

ou

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

(II)

ou

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

(III)

dans lesquelles $R^1$, $R^2$, A, B, C, X et Y sont définis comme dans la revendication 1 et avec la même réserve que celle indiquée dans la revendication 1.

6. Procédé selon la revendication 5 dans lequel $R^1$ est choisi parmi un atome d'hydrogène ou un groupe alcanoyle en $C_1$—$C_3$; $R^2$ est $NR^3R^4$ ou $OR^3$; $R^3$ et $R^4$ sont choisis chacun parmi un atome d'hydrogène et un groupe alkyle en $C_1$—$C_3$; A est un groupe tyrosyle, D-tyrosyle ou histidyle; B est un groupe alanyle ou D-alanyle; C est un groupe aspartyle ou D-aspartyle; X est un groupe asparaginyle ou D-asparaginyle; Y est un groupe norleucyle ou méthionyle; avec la réserve précitée; et ses sels pharmaceutiquement acceptables.

7. Procédé selon la revendication 5 dans lequel le peptide répond à la formule I; $R^1$ est un atome d'hydrogène ou un groupe acétyle; $R^2$ est $NH_2$; A est un groupe tyrosyle, D-tyrosyle ou histidyle; B est un groupe alanyle ou D-alanyle; C est un groupe aspartyle ou D-aspartyle; X est un groupe asparaginyle ou D-asparaginyle; Y est un groupe norleucyle ou méthionyle; avec la réserve précitée; et ses sels pharmaceutiquement acceptables.

8. Procédé selon la revendication 5, dans lequel le peptide est:

$[D-Ala^2]$-hpGRF(1—29)-$NH_2$;
Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables;
Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;
His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;
Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;
Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables;
Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-$NH_2$ et ses sels pharmaceutiquement acceptables; ou
Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-$NH_2$ et ses sels pharmaceutiquement acceptables.

9. Procédé pour la préparation d'un peptide de formule:

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-R^2;$$

(I)

ou

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Gln-Glu-Arg-Gly-Ala-R^2$$

(II)

ou

$$R^1-A-B-C-Ala-Ile-Phe-Thr-X-Ser-Tyr-Arg-Lys-Val-Leu-$$
$$Gly-Gln-Leu-Ser-Ala-Arg-Lys-Leu-Leu-Gln-Asp-Ile-Y-Ser-$$
$$Arg-Gln-Gln-Gly-Glu-Ser-Asn-Glu-Glu-Arg-Gly-Ala-Arg-$$
$$Ala-Arg-Leu-R^2;$$

(III)

dans lesquelles $R^1$, $R^2$, A, B, C, X et Y sont définis comme dans la revendication 1, avec la même réserve que celle mentionnée dans la revendication 1, et leurs sels pharmaceutiquement acceptables; ledit procédé comprenant: la fixation d'un amino-acide protégé C-terminal à une résine en présence d'un agent de couplage; le lavage avec un solvant inerte; la déprotection en présence d'un acide; la répitition de cette séquence avec les amino-acides protégées suivants, comme défini dans les formules (I), (II) et (III),

graduellement à partir de l'extrémité C-terminale du peptide; et finalement la séparation du peptide de la résine en présence d'un acide pour fournir un des peptides de formules (I), (II) ou (III), dans lequel ladite résine est une résine de benzhydrylamine (BHA) ou une résine de p-méthylbenzhydrylamine (p-Me-BHA); ou la séparation du peptide de la résine avec l'ammoniac, une alkylamine ou une dialkylamine et la déprotection avec un acide pour fournir un des peptides de formules (I), (II) ou (III), où ladite résine est une résine chlorométhylée ou hydroxyméthylée; ou la séparation du peptide de la résine en présence d'un acide pour fournir un des peptides de formules (I), (II) ou (III), où ladite résine est une résine chlorométhylée ou hydroxyméthylée; ou la séparation du peptide de la résine en présence d'une et d'un alcool R³OH, suivie d'un traitement acide pour fournir un des peptides de formules (I), (II) ou (III), où R³ est un groupe alkyle à chaîne droite ou ramifiée contenant un à six atomes de carbone et ladite résine est une résine chlorométhylée ou hydroxyméthylée.

10. Procédé pour la préparation d'un peptide selon la revendication 9, dans lequel ledit peptide est caractérisé par la formule:

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables;
Tyr-Ala-D-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables;
Ac-His-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables;
Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables;
Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables;
Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables;
Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ et ses sels pharmaceutiquement acceptables;
His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ et ses sels pharmaceutiquement acceptables;
Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ et ses sels pharmaceutiquement acceptables;
Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ et ses sels pharmaceutiquement acceptables;
Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH₂ et ses sels pharmaceutiquement acceptables; ou
Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables.

11. Peptide caractérisé par la formule:

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-leu-Gln-Asp-Ile-Met-Ser-Arg-NH₂ et ses sels pharmaceutiquement acceptables; ou
Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables.

12. Procédé pour la préparation d'un peptide de formule:

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables ou
Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH₂ et ses sels pharmaceutiquement acceptables,

ledit procédé comprenant: la fixation d'un amino-acide protégé C-terminal à une résine en présence d'un agent de couplage; le lavage avec un solvant inerte; la déprotection en présence d'un acide; la répitition de cette séquence avec les amino-acides protégées suivants, comme défini dans les peptides de formules ci-dessus graduellement à partir de l'extrémité C-terminale du peptide; et finalement la séparation du peptide de la résine en présence d'un acide pour fournir les peptides de formules ci-dessus, dans lequel ladite résine est une résine de benzhydrylamine (BHA) ou une résine de p-méthylbenzhydrylamine (p-Me-BHA); ou la séparation du peptide de la résine avec l'ammoniac, une alkylamine ou une dialkylamine et la déprotection avec un acide pour fournir les peptides de formules ci-dessus, où ladite résine est une résine chlorométhylée ou hydroxyméthylée; ou la séparation du peptide de la résine en présence d'un acide pour fournir les peptides de formules ci-dessus où ladite résine est une résine chlorométhylée ou hydroxyméthylée; ou la séparation du peptide de la résine en présence d'une base et d'un alcool $R^3OH$, suivie d'un traitement acide pour fournir les peptides de formules ci-dessus, où $R^3$ est un groupe alkyle à chaîne droite ou ramifiée contenant un à six atomes de carbone et ladite résine est une résine chlorométhylée ou hydroxyméthylée.

**Revendications pour l'Etat contractant: AT**

1. Procédé pour la préparation d'un peptide de formule:

```
R1-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-R2;
```

(I)

ou

```
R1-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-Gln-Gln-Gly-Glu-Ser-Asn-Gln-Glu-Arg-Gly-Ala-R2
```

(II)

ou

```
R1-A-B-C-Ala-Ile-Phe-Thr-X-Ser-Tyr-Arg-Lys-Val-Leu-
Gly-Gln-Leu-Ser-Ala-Arg-Lys-Leu-Leu-Gln-Asp-Ile-Y-Ser-
Arg-Gln-Gln-Gly-Glu-Ser-Asn-Glu-Glu-Arg-Gly-Ala-Arg-
Ala-Arg-Leu-R2;
```

(III)

dans laquelle $R^1$ est un atome d'hydrogène ou un groupe alcanoyle en $C_1$—$C_6$ à chaîne droite ou ramifiée; $R^2$ est $NR^3R^4$ ou $OR^3$; $R^3$ et $R^4$ sont choisis dans le groupe constitué par un atome d'hydrogène et un groupe alkyle à chaîne droite ou ramifiée contenant un à six atomes de carbone; A est un groupe tyrosyle, D-tyrosyle ou histidyle; B est un groupe alanyle ou D-alanyle; C est un groupe aspartyle ou D-aspartyle; X est un groupe asparaginyle ou D-asparaginyle; Y est un groupe norleucyle ou méthionyle; sous réserve que B et/ou C et/ou X soient un D-amino-acide; et leurs sels pharmaceutiquement acceptables; ledit procédé comprenant: la fixation d'un amino-acide protégé C-terminal à une résine en présence d'un agent de couplage; le lavage avec un solvant inerte; la déprotection en présence d'un acide; la répitition de cette séquence avec les amino-acides protégées suivants, comme défini dans les formules (I), (II) et (III), graduellement à partir de l'extrémité C-terminale du peptide; et finalement la séparation du peptide de la résine en présence d'un acide pour fournir un des peptides de la formules (I), (II) ou (III), dans lequel ladite résine est une résine de benzhydrylamine (BHA) ou une résine de p-méthylbenzhydrylamine (p-Me-BHA); ou la séparation du peptide de la résine avec l'ammoniac, une alkylamine ou une dialkylamine et la déprotection avec un acide pour fournir un des peptides de formules (I), (II) ou (III), où ladite résine est une résine chlorométhylée ou hydroxyméthylée; ou la séparation du peptide de la résine en présence d'un acide pour fournir un des peptides de formules (I), (II) ou (III), où ladite résine est une résine chlorométhylée ou hydroxyméthylée; ou la séparation du peptide de la résine en présence d'une base et d'un alcool $R^3OH$, suivie d'un traitement acide pour fournir un des peptides de formules (I), (II) ou (III), où $R^3$ est un groupe alkyle à chaîne droite ou ramifiée contenant un à six atomes de carbone et ladite résine est une résine chlorométhylée ou hydroxyméthylée.

2. Procédé pour la préparation d'un peptide selon la revendication 1, dans lequel ledit peptide est caractérisé par la formule:

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables;

Tyr-Ala-D-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables;

Ac-His-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables;

Ac-D-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables;

Tyr-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables;

Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ et ses sels pharmaceutiquement acceptables;

His-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ et ses sels pharmaceutiquement acceptables;

Tyr-D-Ala-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ et ses sels pharmaceutiquement acceptables;

Tyr-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ et ses sels pharmaceutiquement acceptables;

Tyr-D-Ala-D-Asp-Ala-Ile-Phe-Thr-D-Asn-Ser-Tyr-Arg-Lys-Val-Leu-Gly-Gln-Leu-Ser-Ala-Arg-Lys-Leu-Leu-Gln-Asp-Ile-Nle-Ser-Arg-NH$_2$ et ses sels pharmaceutiquement acceptables; ou

Ac-Tyr-D-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables.

3. Procédé pour la préparation d'un peptide de formule:

D-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Ieu-Gln-Asp-Ile-Met-Ser-Arg-NH$_2$ et ses sels pharmaceutiquement acceptables  ou

Ac-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-NH$_2$ et ses sels pharmaceutiquement acceptables.

ledit procédé comprenant: la fixation d'un amino-acide protégé C-terminal à une résine en présence d'un agent de couplage; le lavage avec un solvant inerte; la déprotection en présence d'un acide; la répitition de cette séquence avec les amino-acides protégées suivants, comme défini dans les peptides de formules ci-dessus graduellement à partir de l'extrémité C-terminale du peptide; et finalement la séparation du peptide de la résine en présence d'un acide pour fournir les peptides de formules ci-dessus, dans lequel ladite résine est une résine de benzhydrylamine (BHA) ou une résine de p-méthylbenzhydrylamine (p-Me-BHA); ou la séparation du peptide de la résine avec l'ammoniac, une alkylamine ou une dialkylamine et la déprotection avec un acide pour fournir les peptides de formules ci-dessus, où ladite résine est une résine chlorométhylée ou hydroxyméthylée; ou la séparation du peptide de la résine en présence d'un acide pour fournir les peptides de formules ci-dessus où ladite résine est une résine chlorométhylée ou hydroxyméthylée; ou la séparation du peptide de la résine en présence d'une base et d'un alcool R$^3$OH, suivie d'un traitement acide pour fournir les peptides de formules ci-dessus, où R$^3$ est un groupe alkyle à chaîne droite ou ramifiée contenant un à six atomes de carbone et ladite résine est une résine chlorométhylée ou hydroxyméthylée.